# DEMANDE DE BREVET EUROPEEN

(11) **EP 1 092 426 A1**
(43) Date de publication de la demande: **18.04.2001**
(21) Numéro de dépôt: 00402525.0
(22) Date de dépôt: 13.09.2000
(51) Int. Cl.: A61K 7/48

(54) **Utilisation de complexes inorganiques-organiques dans une composition**

(30) Priorité: 28.09.1999 FR 9912057
(71) Demandeur: L'OREAL, 75008 Paris (FR)
(72) Inventeur: Philippe, Michel, 91320 Wissous (FR); Cohen, Catherine, 75012 Paris (FR)
(74) Mandataire: Lhoste, Catherine

(57) **Abrégé**

La présente invention se rapporte à un procédé pour diminuer l'irritation de la peau, cette irritation pouvant notamment être due à certains produits appliqués sur la peau ou à l'environnement, en appliquant des complexes inorganiques-organiques. Elle a trait également à une composition comprenant ces complexes.

## Description

La présente invention se rapporte à un procédé pour diminuer l'irritation de la peau, cette irritation pouvant notamment être due à certains produits appliqués sur la peau ou à l'environnement, en appliquant des complexes inorganiques-organiques. Elle a trait également à une composition comprenant ces complexes.

De nombreux composés sont utilisés en cosmétique pour améliorer l'apparence de la peau, pour embellir la peau, pour traiter des désordres cutanés ou pour rendre la peau moins sèche ou plus souple. Malheureusement, certains de ces composés peuvent provoquer une irritation lorsqu'ils sont appliqués sur la peau ou les muqueuses.

Il est connu d'utiliser des dérivés de l'acide salicylique comme agent kératolytique pour traiter l'acné et comme agent d'antivieillissement dans des compositions cosmétiques et/ou dermatologiques. Ainsi, les documents FR-A-2 581 542 et EP-A-378 936 décrivent de tels dérivés.

Les dérivés d'acide salicylique sont d'un grand intérêt, étant donné leurs effets biologiques sur la peau, notamment sur les principaux signes cliniques du vieillissement cutané que sont les ridules et rides, la désorganisation du "grain" de la peau, la modification du teint de la peau et la perte de fermeté et de tonicité de la peau. Cependant, l'utilisation de ces dérivés pose un problème dans la mesure où ils peuvent provoquer des picotements, des démangeaisons, des tiraillements après leur application, pouvant conduire à un important inconfort. L'utilisation de ces composés pour les utilisateurs, et plus particulièrement ceux à peau sensible, est donc souvent rédhibitoire. La peau sensible et les signes caractéristiques des peaux sensibles ont été définis dans le brevet EP 680749.

Il en est de même pour les agents exfoliants tels que les hydroxy-acides. Il a été préconisé pour ces composés, dans le brevet EP 413528, de les associer à des composés amphotères pour diminuer l'inconfort qu'ils peuvent provoquer.

Il est également largement décrit que certains utilisateurs de produits cosmétiques comprenant des tensio-actifs, des conservateurs, des parfums, des solvants ou des propulseurs se plaignent d'avoir la peau qui les irrite après application sur leur peau de l'un de ces produits.

Mais du fait de leur caractère irritant, ces produits sont généralement utilisés en des doses très faibles. L'utilisation à faible quantité de ces produits peut alors s'avérer peu avantageuse par rapport à l'utilisation d'autres produits moins actifs, mais qui sont utilisés en plus grande quantité, car moins ou pas irritants.

Il est également décrit que certaines personnes présentent une peau irritée lorsque leur peau a été exposée à un environnement chimique ou physique particulier ou inhabituel, notamment lorsqu'elle est exposée à des variations importantes de température, au vent ou à un environnement très pollué. Ainsi, la peau peut réagir par des démangeaisons, des tiraillements ou des picotements.

Ainsi, l'irritation peut se caractériser par des picotements, des démangeaisons ou des tiraillements, ce qui peut conduire à un important inconfort.

Les documents WO-A-96/19184, WO-A-96/19182 et WO-A-96/19228 décrivent l'utilisation de sels divalents de strontium, manganèse, magnésium et calcium, solubles dans l'eau pour diminuer l'irritation de composés ayant un effet irritant, notamment d'exfoliants tels que les hydroxy-acides.

La demanderesse a découvert de façon surprenante que des complexes inorganiques-organiques permettent de diminuer l'irritation induite notamment par certains produits appliqués sur la peau ou par l'environnement.

L'invention a donc pour premier objet une composition comprenant, dans un milieu physiologiquement acceptable, au moins un composé de formule (I) : 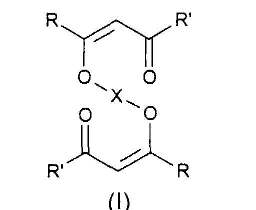 dans laquelle :
R, identique ou différent, représente un radical alkyle ou perfluoroalkyle, linéaire ou ramifiée, saturé ou insaturé, éventuellement hydroxylé, ayant de 1 à 12 atomes de carbone ;
R', identique ou différent, représente un radical alkyle, alkyloxy ou perfluoroalkyle, linéaire ou ramifié, saturé ou insaturé, éventuellement hydroxylé, ayant de 1 à 12 atomes de carbone ;
X est un élément choisi parmi le magnésium, le strontium, le baryum, le zinc, le manganèse, le palladium ou le cuivre,
ce composé se trouvant sous forme hydratée ou non. Ce composé est en particulier un complexe organique-inorganique.

La présente invention concerne aussi un procédé cosmétique de traitement cosmétique, caractérisé en ce qu'il met en oeuvre la composition cosmétique suivant l'invention. Ainsi, ce procédé comprend l'application sur la peau, les fibres kératiniques, les ongles ou les muqueuses de la composition cosmétique selon l'invention.

L'invention a encore pour objet l'utilisation d'au moins un composé de formule (I) : 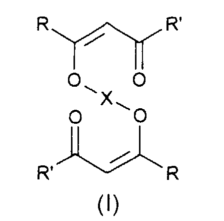 dans laquelle :
R, identique ou différent, représente un radical alkyle ou perfluoroalkyle, linéaire ou ramifiée, saturé ou insaturé, éventuellement hydroxylé, ayant de 1 à 12 atomes de carbone ;
R', identique ou différent, représente un radical alkyle, alkyloxy ou perfluoroalkyle, linéaire ou ramifié, saturé ou insaturé, éventuellement hydroxylé, ayant de 1 à 12 atomes de carbone ;
X est un élément de la colonne IIA de la classification périodique (version CAS), le zinc, le manganèse, le palladium ou le cuivre,
ce composé se trouvant sous forme hydratée ou non,
dans une composition cosmétique ou pour la fabrication d'une composition dermatologique ou pharmaceutique comme agent diminuant l'irritation cutanée. Ce composé joue notamment le rôle d'agent apaisant et peut être utilisé comme produit cosmétique.

Selon la présente invention, le radical alkyle linéaire ou ramifié ayant de 1 à 12, atomes de carbone est notamment choisi parmi un radical méthyle, éthyle, propyle, isopropyle, butyle, tertiobutyle, hexyle, 2-éthyl-hexyle, octyle, nonyle, dodécyle, 2-méthylbutyle, 2-méthylpentyle, 1-méthylhexyle et 3-méthylheptyle.

De préférence, R est un radical alkyle ou perfluoroalkyle, linéaire ou ramifié, saturé, ayant de 1 à 6 atomes de carbone. En outre, R' est avantageusement un radical alkyle ou perfluoroalkyle, alkyloxy ou perfluoroalkyle, linéaire ou ramifié, saturé, ayant de 1 à 6 atomes de carbone.

De préférence, les substituants R et R' sont linéaires et/ou saturés.

En particulier, les substituants R et R' comprennent au plus deux atomes de carbone de différence l'un par rapport à l'autre.

Ces composés de formule (I) sont insolubles dans l'eau, contrairement aux composés décrits dans WO-A-96/19184, WO-A-96/19182 et WO-A-96/19228. Leur propriété de diminuer l'irritation notamment induite par certains produits appliqués sur la peau qui sont généralement hydrophiles, tels que les hydroxy-acides, est ainsi d'autant plus surprenante.

Comme dérivés utilisables selon l'invention on peut citer le strontium 2,4-pentanedionate, le bis (1,1,1,2,2,3,3-heptafluoro-7,7-dimethyl-4,6-octanedionato) strontium hydrate, le bis (2,2,6,6-tetraméthyl-3,5-heptanedionato) strontium hydrate, le strontium hexafluoroacétylacétonate, le calcium 2,4-pentanedionate, le bis (1,1,1,2,2,3,3-heptafluoro-7,7-diméthyl-4,6-octanedionato) calcium, le calcium hexafluoroacétylacétonate dihydrate et le calcium 2,2,6,6-tetraméthyl-3,5-heptane-dionate et leurs mélanges.

Certains de ces composés ont été notamment décrits en tant que catalyseurs de réactions chimiques ou comme réactifs de procédés d'obtention de composés minéraux, tels que des supraconducteurs (WO91/13051 ou "Preparation and properties of .beta-diketone and beta-keto ester complexes of bismuth (III), strontium (I) and calcium (I)" de Doi Masura *et al*. dans Nippon Kagaku Kaishi (1995), (10), 802-8).

De manière préférentielle les substituants R et R' sont des radicaux méthyle.

Avantageusement X est choisi parmi le strontium, le calcium, le magnésium, le manganèse, le baryum, le cuivre, le zinc ou le palladium et plus spécialement choisi parmi le strontium, le calcium le cuivre ou le zinc.

La composition selon l'invention est destinée en particulier à un usage topique sur la peau, les fibres kératiniques, les ongles ou les muqueuses, en particulier la composition est une composition cosmétique et/ou dermatologique.

Par milieu physiologiquement acceptable, on entend un milieu compatible avec la peau, les muqueuses (y compris l'intérieur des paupières et les lèvres), les ongles et/ou les fibres kératiniques (cheveux et/ou poils). En outre, ce milieu physiologiquement acceptable ne comprend pas de produit réactionnel secondaire ou résiduel car les composés de formule (I) résultent d'un procédé de synthèse propre.

En effet, ces composés sont obtenus suivant un procédé mettant en oeuvre un cation activé carbonaté ou hydroxylé, qui va réagir avec une β dicétone (le composé organique de départ). Les produits réactionnels secondaires formés durant la réaction sont du gaz carbonique ou de l'eau et s'éliminent du milieu réactionnel notamment sous forme de gaz ou sont facilement éliminés après réaction (en particulier avec les eaux de lavage). En outre, si ces produits réactionnels secondaires restent présents à l'état de trace, ils sont non toxiques et /ou non réactifs vis-à-vis des composés de formule (I).

Ainsi, la présente invention concerne également une composition selon l'invention dans laquelle le composé de formule (I) résulte de la réaction d'une β-dicétone et d'un cation activé hydroxylé ou carbonaté.

Les composés de formule (I) selon l'invention sont avantageusement présents en une quantité efficace pour assurer le résultat escompté, c'est-à-dire diminuer ou supprimer l'irritation. Ils peuvent être par exemple présents dans une composition selon l'invention en une quantité pondérale allant de 0,001 à 30 %, de préférence de 0,01 à 20 % et mieux de 0,1 à 10 % du poids total de la composition.

Les compositions contenant un composé selon l'invention peuvent se présenter sous toutes les formes galéniques normalement utilisées et notamment celles appropriées pour une application topique, par exemple sous forme d'une solution huileuse, d'un gel huileux, d'un produit anhydre liquide, pâteux ou solide, d'une émulsion du type eau-dans-huile (E/H), huile-dans-eau (H/E) ou multiple (E/H/E ou H/E/H), d'une microémulsion ou d'une dispersion de vésicules de type ionique et/ou non ionique. Ces compositions sont préparées selon les méthodes usuelles.

Comme spécifié précédemment, les composés de formule (I) se trouvent plus particulièrement dans la phase grasse des compositions selon l'invention.

Ces compositions peuvent être plus ou moins fluides et avoir l'aspect d'une crème blanche ou colorée, d'une pommade, d'un lait, d'une lotion, d'un sérum, d'une pâte, d'une mousse. Elles peuvent éventuellement être appliquées sur la peau sous forme d'aérosol. Elles peuvent également se présenter sous forme solide, et par exemple sous forme de stick.

Ces compositions peuvent comprendre au moins un adjuvant choisi parmi l'eau, les corps gras, les conservateurs, les gélifiants, les tensioactifs et émulsionnants, les antioxydants, les charges, les solvants, les parfums, les matières colorantes, les filtres, les actifs cosmétiques et/ou dermatologiques tels que les hydratants, les vitamines et les actifs antivieillissement, et leurs mélanges. Les quantités utilisées de ces différents additifs sont celles classiquement utilisées dans le domaine cosmétique et/ou dermatologique.

Les corps gras peuvent être choisis parmi les huiles de synthèse, les huiles d'origine animale, les huiles d'origine végétale, les huiles minérales (huile de vaseline), les huiles de silicone, les huiles fluorées et leurs mélanges. On peut aussi utiliser des alcools gras, des acides gras, des cires.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition.

Lorsque la composition est une solution ou un gel huileux, la phase grasse peut représenter plus de 90 % du poids total de la composition.

Comme émulsionnants utilisables dans l'invention, on peut citer par exemple les éthers oléiques de polyéthylène glycol tels que les produits vendus sous les dénominations BRIJ92 et BRIJ96 par la société ICI.

Les quantités des différents constituants des compositions selon l'invention sont celles classiquement utilisées dans les domaines considérés. La nature des adjuvants et leurs quantités doivent être telles qu'elles ne modifient pas les propriétés des composés de formule (I) selon l'invention.

Les compositions de l'invention peuvent être utilisées comme produit de nettoyage, de protection, de traitement ou de soin et/ou comme produit de maquillage de la peau du visage et/ou du corps, des muqueuses et/ou des fibres kératiniques. Elles constituent notamment des crèmes de traitement ou de soin pour le visage, pour les mains ou pour le corps, (par exemple crèmes de jour, crèmes de nuit, crèmes solaires), des laits corporels de protection ou de soin, des lotions (lotions de nettoyage, lotions solaires), des gels ou mousses pour le soin de la peau, des compositions pour le bain. Elles constituent aussi des produits de maquillage notamment des joues, des lèvres, des cils et des paupières, tels que des fonds de teint, des rouges à lèvre et des fards à paupière : à cet effet, elles peuvent comprendre des matières colorantes et notamment des pigments ou des colorants chimiques.

Elles peuvent être également utilisées pour les cheveux sous forme de solutions, de crèmes, de gels, d'émulsions, ou de mousses et notamment comme compositions pour soins capillaires, tels que shampooings, lotions traitantes, lotions restructurantes pour les cheveux, lotions ou gels antichute, shampooings antiparasitaires, etc

Les compositions selon l'invention peuvent également consister en des préparations solides constituant des savons ou des pains de nettoyage.

La composition comprenant le composé de formule (I) peut comprendre en outre le produit susceptible de provoquer une irritation cutanée.

Dans ce cas, la présente invention concerne également une composition, de préférence cosmétique, caractérisée en ce qu'elle comprend, dans un milieu physiologiquement acceptable, au moins un composé de formule (I) et au moins un produit susceptible de provoquer une irritation cutanée.

La quantité d'au moins un composé de formule (I) selon l'invention est de préférence une quantité suffisante pour que l'effet irritant cutané diminue, voire disparaisse. Ainsi, cette quantité est variable notamment en fonction de la quantité et de la nature du produit à caractère irritant appliqué. Cependant, à titre d'illustration, et tel que spécifié précédemment, une composition selon l'invention peut comprendre au moins un composé de formule (I) en une quantité allant de 0,001 à 30 %, de préférence de 0,01 à 20 % et mieux de 0,1 à 10 % du poids total de la composition.

Dans la composition selon l'invention, la quantité du produit susceptible de provoquer une irritation cutanée peut donc correspondre à une quantité suffisante pour provoquer une irritation cutanée s'il était utilisé seul (sans le composé de formule (I)).

La présente invention présente en effet l'avantage de pouvoir augmenter la quantité d'agents actifs, classiquement à caractère irritant, dans des compositions par rapport à la quantité normalement utilisée, en vue d'une efficacité de ces derniers améliorée. Ainsi, la quantité d'agent actif pouvant être utilisée dans la composition va de 5% à 50% du poids total de la composition. En particulier, on peut utiliser les hydroxyacides jusqu'à 50 % du poids total de la composition, l'acide salicylique ou ses dérivés jusqu'à 10% ou les rétinoïdes jusqu'à 5 %, sans aucune gène pour l'utilisateur.

De nombreux produits appliqués topiquement présentent un caractère irritant, spécialement pour les personnes (utilisateurs) à peaux facilement irritables. Ainsi, ces produits, tel que décrit précédemment, provoquent des picotements, des démangeaisons ou des tiraillements, entraînant un inconfort rédhibitoire pour l'utilisateur.

Les produits susceptibles de provoquer une irritation cutanée sont de préférence choisis parmi des conservateurs, des tensio-actifs, des parfums, des solvants, des propulseurs et des agents actifs et leurs mélanges.

Ainsi, même les produits qui sont considérés comme inertes dans une composition cosmétique peuvent présenter un caractère irritant lorsqu'ils sont appliqués sur la peau, le cuir chevelu, les ongles ou les muqueuses, tels que notamment des conservateurs, des tensio-actifs, des parfums, des solvants ou des propulseurs.

Aussi, des produits étant considérés comme des agents actifs dans des compositions cosmétiques peuvent présenter un caractère irritant lorsqu'ils sont appliqués sur la peau, le cuir chevelu, les ongles ou les muqueuses. On peut parler d'effet secondaire irritant présentés par ces produits, qui sont en particulier des agents actifs, tels que notamment certains filtres solaires, les hydroxy-acides en particulier les α hydroxy-acides (glycolique, lactique, malique, citrique, tartrique, mandélique) et les β hydroxy-acides notamment l'acide salicylique et ses dérivés, les céto-acides en particulier sous forme α et β, les dérivés d'hydroxy- ou de céto-acides notamment sous forme α et β, les rétinoïdes (rétinol et ses esters, rétinal, acide rétinoïque et ses dérivés, rétinoïdes, notamment ceux décrits dans les documents FR-A-2 570 377, EP-A-199 636, EP-A-325 540, EP-A-402 072), les anthralines (dioxyanthranol), les anthranoïdes (par exemple ceux décrits dans le document EP-A- 319028), les peroxydes (notamment le peroxyde de benzoyle), le minoxidil et ses dérivés, les sels de lithium, les antiprolifératifs, tels que le 5-fluorouracyle ou le méthotrexate, certaines vitamines telles que la vitamine D et ses dérivés, la vitamine B9 et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), la capsaïcine, les actifs antipoux (pyréthrine), les agents détergents ioniques et non ioniques et des propigmentants (la dihydroxyacétone, les psoralènes et les méthylangécilines) et leurs mélanges.

Parmi la vitamine D et ses dérivés, on peut citer plus particulièrement la vitamine D3, la vitamine D2, la 1, 25-diOH vitamine D3 (le calcitriol), le calcipotriol, la 1, 24-diOH vitamine D3 (tel que le tacalcitol), la 24, 25-diOH vitamine D3, la 1-OH vitamine D2, la 1, 24-diOH vitamine D2.

Parmi les dérivés de l'acide salicylique, on peut citer plus particulièrement l'acide n-octanoyl-5-salicylique et l'acide n-dodécanoyl-5-salicylique, leurs sels ou leurs esters.

Un autre objet de l'invention concerne l'utilisation d'au moins un composé de formule (I) tel que défini précédemment dans une composition cosmétique non irritante ou dans une composition cosmétique destinée aux personnes à peau sensible.

Les compositions qui comprennent au moins un composé de formule (I) et au moins un agent actif susceptible de provoquer une irritation cutanée se sont révélées particulièrement appropriées pour le soin et/ou le traitement et/ou le maquillage de la peau et/ou des muqueuses et/ou des fibres kératiniques d'être humain, et notamment pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique et/ou pour le traitement de l'acné et/ou pour le traitement des désordres cutanés.

Ainsi, un autre objet de l'invention concerne l'utilisation d'au moins un composé de formule (I) dans une composition cosmétique contenant un agent actif susceptible de provoquer une irritation cutanée et étant destinée au soin de la peau et/ou des muqueuses et/ou des fibres kératiniques d'être humain, et notamment destinée à améliorer l'éclat du teint et/ou lisser la peau du visage et/ou du corps et/ou lutter contre les signes du vieillissement cutané et/ou l'acné.

En outre, un autre objet de l'invention l'invention concerne l'utilisation d'au moins un composé de formule (I) pour la fabrication d'une composition dermatologique ou thérapeutique contenant un agent actif susceptible de provoquer une irritation cutanée et étant destinée à lutter contre les signes du vieillissement cutané et/ou traiter les rides et les ridules de la peau et/ou stimuler le processus de renouvellement épidermique et/ou traiter l'acné et/ou les désordres cutanés.

Par désordres cutanés, on entend en particulier le zona, les brûlures, l'eczéma, la démodécie, l'ulcère cutané, la fibrose, le contrôle des cicatrisations, le psoriasis, les prurits, les dermatites, l'ichtyose, les cors et les verrues.

D'autres caractéristiques et avantages de l'invention ressortiront mieux des exemples qui suivent, donnés à titre illustratif et non limitatif. Les pourcentages sont exprimés en poids, sauf mention contraire.

### Exemple 1 : Lait pour la peau, à activité exfoliante

- acide 5-n-octanoyl-salicylique 0,5 %
- strontium 2,4-pentanedionate 0,5 %
- BRIJ95 2,5 %
- BRIJ92 2,5 %
- Huile de vaseline 30 %
- Eau qsp 100 %

Le lait obtenu est doux, a une bonne propriété exfoliante pour la peau et n'irrite pas la peau. Il peut être utilisé par les personnes à peau sensible.

### Exemple 2 : Test sur peau reconstruite

Le système Episkin est un modèle d'épiderme humain reconstitué *in vitro* produit par la société Episkin. Cet épiderme ne contient qu'un seul type cellulaire : les kératinocytes. Les kératinocytes sont ensemencés sur un équivalent de derme composé de collagènes de différentes types.

Après une période de prolifération des kératinocytes en immersion dans le milieu de culture, le système est surélevé à l'interface air-liquide. Cette étape d'émersion des cultures induit la différenciation des kératinocytes vers le stade terminal de cornéocytes en 13 jours, permettant ainsi la reconstruction in vitro d'un épiderme doté d'une couche basale, d'une couche spineuse, d'une couche granuleuse et d'une couche cornée.

La demanderesse a étudié la cytotoxicité des kératinocytes (test au bromure de 3-(4,5-diMéthylThiazole-2-yl)-2,5-diphénylTétrazolium) après application sur ce modèle de peau reconstruite de différentes compositions à raison de 150mg/cm2 et maintien à l'étuve à 37°C / 5%CO₂ pendant une période de 18 heures. Chaque composition est testée sur deux épidermes de 1,13cm2.

La composition A de base est :
- BRIJ95 2,5 %
- BRIJ92 2,5 %
- Huile de vaseline 30 %
- Eau qsp 100 %
pH =5,5 ± 0,3

Les autres compositions testées correspondent à cette composition A à laquelle est ajouté l'acide 5-n-octanoyl-salicylique, seul ou en mélange avec un composé de formule (I) selon l'invention.

La mesure de la viabilité cellulaire est réalisée immédiatement après rinçage des compositions au "Phosphate Buffer Saline" (PBS).

Ainsi, la composition A n'est pas cytotoxique. L'ajout de 0,3% (p/V) de l'acide 5-n-octanoyl-salicylique à cette composition la rend cytotoxique.

Les différentes compositions testées donnent les résultats rassemblés dans le tableau ci-dessous.

| Composé de formule (I) ajouté à la composition A | Concentration en % (p/V) du composé de formule (I) ajouté | Concentration en % (p/V) de l'acide 5-n-octanoylsalicylique ajouté à la composition A | % de viabilité cellulaire |
|---|---|---|---|
| - | 0 | 0,5 | 7,8 |
| Strontium 2,4-pentanedionate | 0,54 | 0,5 | 95,5 |
| Strontium 2,4-pentanedionate | 0,8 | 0,75 | 125,6 |
| Strontium 2,4-pentanedionate | 1,08 | 1 | 113,4 |
| p/V : poids/volume | | | |

L'ajout d'un composé de formule (I) à la composition A en présence de 0.5, 0.75 ou 1 % d'acide 5-n-octanoyl-salicylique ne rend pas la composition A cytotoxique.

## Revendications

1. Composition comprenant, dans un milieu physiologiquement acceptable, au moins un composé de formule (I) : 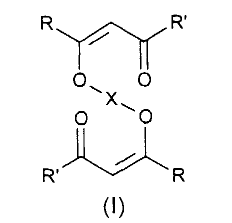 dans laquelle :
R, identique ou différent, représente un radical alkyle ou perfluoroalkyle, linéaire ou ramifiée, saturé ou insaturé, éventuellement hydroxylé, ayant de 1 à 12 atomes de carbone ;
R', identique ou différent, représente un radical alkyle, alkyloxy ou perfluoroalkyle, linéaire ou ramifié, saturé ou insaturé, éventuellement hydroxylé, ayant de 1 à 12 atomes de carbone ;
X est un élément choisi parmi le magnésium, le strontium, le baryum, le zinc, le manganèse, le palladium ou le cuivre,
ce composé se trouvant sous forme hydratée ou non.

2. Composition selon la revendication 1, caractérisée en ce que, dans le composé de formule (I), R est un radical alkyle ou perfluoroalkyle, linéaire ou ramifié, saturé, ayant de 1 à 6 atomes de carbone.

3. Composition selon l'une quelconque des revendications 1 et 2, caractérisée en ce que, dans le composé de formule (I), R' est un radical alkyle, alkyloxy ou perfluoroalkyle, linéaire ou ramifié, saturé, ayant de 1 à 6 atomes de carbone.

4. Composition selon l'une quelconque des revendications précédentes, caractérisée en ce que, dans le composé de formule (I), les substituants R et R' sont linéaires.

5. Composition selon l'une quelconque des revendications précédentes, caractérisée en ce que, dans le composé de formule (I), les substituants R et R' sont saturés.

6. Composition selon l'une quelconque des revendications précédentes, caractérisée en ce que, dans le composé de formule (I), les substituants R et R' comprennent au plus deux atomes de carbone de différence l'un par rapport à l'autre.

7. Composition selon l'une quelconque des revendications précédentes, caractérisée en ce que, dans le composé de formule (I), X est choisi parmi le strontium, le cuivre ou le zinc.

8. Composition selon l'une quelconque des revendications précédentes, caractérisée en ce que, dans le composé de formule (I), R et R' sont des radicaux méthyle.

9. Composition selon l'une quelconque des revendications 1 à 7, caractérisée en ce que le composé de formule (I) est choisi parmi le strontium 2,4-pentanedionate, le bis (1,1,1,2,2,3,3-heptafluoro-7,7-dimethyl-4,6-octanedionato) strontium hydrate, le bis (2,2,6,6-tetraméthyl-3,5-heptanedionato) strontium hydrate, le strontium hexafluoroacétylacétonate et leurs mélanges.

10. Composition selon l'une quelconque des revendications précédentes, caractérisée en ce qu'elle comprend au moins un produit susceptible de provoquer une irritation cutanée, le composé de formule (I) étant présent dans la composition en une quantité suffisante pour diminuer ou supprimer la dite irritation.

11. Composition selon l'une quelconque des revendications précédentes, caractérisée en ce que le composé de formule (I) est en une quantité pondérale allant de 0,001 à 30%, de préférence de 0,01 à 20 % et mieux de 0,1 à 10 % du poids total de la composition.

12. Composition selon l'une quelconque des revendications 10 et 11, caractérisée en ce que le produit susceptible de provoquer une irritation cutanée est choisi parmi les conservateurs, les tensio-actifs, des parfums, les solvants, les propulseurs, les agents actifs et leurs mélanges.

13. Composition selon la revendication 12, caractérisée en ce que l'agent actif est choisi parmi les filtres solaires, les hydroxy-acides, les céto-acides, les dérivés d'hydroxy- ou de céto-acides, les rétinoïdes, les anthralines, les anthranoïdes, les peroxydes, le minoxidil et ses dérivés, les sels de lithium, les antiprolifératifs, certaines vitamines, les teintures ou colorants capillaires, les solutions alcooliques parfumantes, les agents antitranspirants, les actifs dépilatoires ou de permanentes, les dépigmentants, la capsaïcine, les actifs antipoux, les agents détergents ioniques et non ioniques et des propigmentants.

14. Composition selon la revendication 12 ou 13, caractérisée en ce que l'agent actif est choisi parmi les α et β hydroxy-acides, les α et β céto-acides, les dérivés α et β hydroxy-ou α et β céto-acides, le 5-fluorouracyle, le méthothrexate, la vitamine B9 et ses dérivés, la vitamine D et ses dérivés

15. Composition selon l'une quelconque des revendications 12 à 14, caractérisée en ce que l'agent actif est choisi parmi les β hydroxy-acides.

16. Composition selon l'une quelconque des revendications 12 à 15, caractérisée en ce que l'agent actif est l'acide salicylique et ses dérivés.

17. Composition selon l'une quelconque des revendications 12 à 16, caractérisée en ce que l'agent actif est choisi parmi l'acide n-octanoyl-5-salicylique et l'acide n-dodécanoyl-5-salicylique, leurs sels ou leurs esters.

18. Composition selon l'une quelconque des revendications 12 à 17, caractérisée en ce que l'agent actif est utilisé en une quantité comprise entre 5% et 50% du poids total de la composition.

19. Composition selon l'une quelconque des revendications précédentes dans laquelle le composé de formule (I) résulte de la réaction d'une β-dicétone et d'un cation activé hydroxylé ou carbonaté.

20. Procédé de traitement cosmétique, caractérisé en ce qu'il comprend l'application sur la peau, les fibres kératiniques, les ongles ou les muqueuses de la composition cosmétique selon l'une quelconque des revendications précédentes.

21. Utilisation d'au moins un composé de formule (I) : 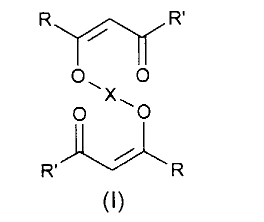 dans laquelle :
R, identique ou différent, représente un radical alkyle ou perfluoroalkyle, linéaire ou ramifiée, saturé ou insaturé, éventuellement hydroxylé, ayant de 1 à 12 atomes de carbone ;
R', identique ou différent, représente un radical alkyle, alkyloxy ou perfluoroalkyle, linéaire ou ramifié, saturé ou insaturé, éventuellement hydroxylé, ayant de 1 à 12 atomes de carbone ;
X est un élément de la colonne IIA de la classification périodique, le zinc, le manganèse le palladium ou le cuivre,
ce composé se trouvant sous forme hydratée ou non,
dans une composition cosmétique ou pour la fabrication d'une composition dermatologique ou pharmaceutique comme agent diminuant l'irritation cutanée.

22. Utilisation selon la revendication 21, caractérisée en ce que, dans le composé de formule (I), R est un radical alkyle ou perfluoroalkyle, linéaire ou ramifié, saturé, ayant de 1 à 6 atomes de carbone.

23. Utilisation selon l'une quelconque des revendications 21 et 22, caractérisée en ce que, dans le composé de formule (I), R' est un radical alkyle, alkyloxy ou perfluoroalkyle, linéaire ou ramifié, saturé, ayant de 1 à 6 atomes de carbone.

24. Utilisation selon l'une quelconque des revendications 21 à 23, caractérisée en ce que, dans le composé de formule (I), les substituants R et R' sont linéaires.

25. Utilisation selon l'une quelconque des revendications 21 à 24, caractérisée en ce que, dans le composé de formule (I), les substituants R et R' sont saturés.

26. Utilisation selon l'une quelconque des revendications 21 à 25, caractérisée en ce que, dans le composé de formule (I), les substituants R et R' comprennent au plus deux atomes de carbone de différence l'un par rapport à l'autre.

27. Utilisation selon l'une quelconque des revendications 21 à 26, caractérisée en ce que, dans le composé de formule (I), X est choisi parmi le strontium, le calcium, le magnésium, le manganèse, le baryum, le cuivre, le zinc ou le palladium.

28. Utilisation selon l'une quelconque des revendications 21 à 27, caractérisée en ce que, dans le composé de formule (I), R et R' sont des radicaux méthyle.

29. Utilisation selon l'une quelconque des revendications 21 à 27, caractérisée en ce que le composé de formule (I) est choisi parmi le strontium 2,4-pentanedionate, le bis (1,1,1,2,2,3,3-heptafluoro-7,7-dimethyl-4,6-octanedionato) strontium hydrate, le bis (2,2,6,6-tetraméthyl-3,5-heptanedionato) strontium hydrate, le strontium hexafluoroacétylacétonate, le calcium 2,4-pentanedionate, le bis (1,1,1,2,2,3,3-heptafluoro-7,7-diméthyl-4,6-octanedionato) calcium, le calcium hexafluoroacétylacétonate dihydrate et le calcium 2,2,6,6-tetraméthyl-3,5-heptane-dionate et leurs mélanges.

30. Utilisation d'au moins un composé de formule (I) tel que défini dans l'une quelconque des revendications 21 à 29, dans une composition cosmétique non irritante.

31. Utilisation d'au moins un composé de formule (I) tel que défini l'une quelconque des revendications 21 à 29, dans une composition cosmétique destinée aux personnes à peau sensible.

32. Utilisation d'au moins un composé de formule (I) tel que défini l'une quelconque des revendications 21 à 31, dans une composition cosmétique contenant un agent actif susceptible de provoquer une irritation cutanée et étant destinée au soin de la peau et/ou des muqueuses et/ou des fibres kératiniques d'être humain, et notamment destinée à améliorer l'éclat du teint et/ou lisser la peau du visage et/ou du corps et/ou lutter contre les signes du vieillissement cutané et/ou l'acné.

33. Utilisation d'au moins un composé de formule (I) tel que défini l'une quelconque des revendications 21 à 31, pour la fabrication d'une composition dermatologique ou thérapeutique contenant un agent actif susceptible de provoquer une irritation cutanée et étant destinée à lutter contre les signes du vieillissement cutané et/ou traiter les rides et les ridules de la peau et/ou stimuler le processus de renouvellement épidermique et/ou traiter l'acné et/ou les désordres cutanés.

34. Utilisation selon la revendication 33 caractérisée en ce que les désordres cutanés sont le zona, les brûlures, l'eczéma, la démodécie, l'ulcère cutané, la fibrose, le contrôle des cicatrisations, le psoriasis, les prurits, les dermatites, l'ichtyose, les cors et les verrues.
